Europäisches Patentamt

European Patent Office

Office européen des brevets

(19)

(11) Veröffentlichungsnummer: **0 249 005**
**A2**

(12) # EUROPÄISCHE PATENTANMELDUNG

(21) Anmeldenummer: **87105424.3**

(22) Anmeldetag: **11.04.87**

(51) Int. Cl.4: **C07C 69/753** , C07C 67/04

(30) Priorität: **12.06.86 DE 3619797**

(43) Veröffentlichungstag der Anmeldung:
**16.12.87 Patentblatt 87/51**

(84) Benannte Vertragsstaaten:
**BE CH DE FR GB IT LI NL**

(71) Anmelder: **HÜLS AKTIENGESELLSCHAFT**
**Patentabteilung / PB 15 - Postfach 13 20**
**D-4370 Marl 1(DE)**

(72) Erfinder: **Kleine-Homann, Walter, Dr.**
**Buchenallee 14**
**D-4408 Dülmen(DE)**

(54) **Verfahren zur Herstellung von Dicyclopentenylestern.**

(57) Bei der Herstellung von Dicyclopentenylestern durch Addition von Carbonsäuren an Dicyclopentadien setzt man lonenaustauscherharze als Katalysatoren ein. Man erhält bei hohen Umsätzen eine hohe Selektivität. Die Wasserkonzentration im Reaktoreinsatz soll 0,5 bis 15 % betragen. Durch Zugabe der korrespondierenden Anhydride vor bzw. bei der destillativen Aufarbeitung kann man die Bildung von schwer abtrennbaren, störenden Zwischenläufen stark reduzieren.

EP 0 249 005 A2

## Verfahren zur Herstellung von Dicyclopentenylestern

Die Erfindung betrifft ein Verfahren zur Herstellung von Dicyclopentenylestern bei hohen Umsätzen und Selektivitäten.

Durch Addition von Carbonsäuren wie Essigsäure oder Propionsäure an Dicyclopentadien werden die entsprechenden Ester erhalten, die für die Riechstoffindustrie wichtige Ausgangsmaterialien darstellen.

Während die Umsetzung mit Ameisensäure zum entsprechenden Formiat autokatalytisch verläuft (Chem. Rev. 69, 1969, Seiten 673 bis 674) werden bei den Reaktionen zu den höheren Homologen Katalysatoren eingesetzt.

Als Katalysatoren sind stark saure Ionenaustauscherharze wie Lewatit SC 104 (EP-PS 0 102 366), Schwefelsäure, Perchlorsäure, Bortrifluorid oder eine Mischung aus Bortrifluorid/Phosphorsäure (US-PS 2 814 639) geeignet.

Für den Einsatz als Riechstoffvorprodukt stellt die Reinigung der Rohprodukte ein besonderes Problem dar. Beispielsweise werden sie häufig vor der Destillation einer wiederholten Wäsche unterworfen und danach aufwendig rektifiziert, um anschließend eventuell nochmals einer Wasserdampfdestillation unterzogen zu werden. Durch Einsatz von Ionentauschern konnte zwar eine Reduzierung der Wäschen bewirkt werden, nachteilig für die Rektifikation ist jedoch die unbefriedigende Selektivität. Durch Anwesenheit von geruchsintensiven, destillativ schwer abtrennbaren Zwischenläufen wird die destillative Aufarbeitung sehr erschwert, wobei z. B. die Destillationszeit verlängert wird. Durch die Verlängerung der Destillationszeit bei den erforderlichen hohen Temperaturen erfolgt eine partielle Zersetzung des Wertproduktes.

Es besteht daher ein Interesse an einem Verfahren, das bei hohen Umsätzen und Selektivitäten zu einem Rohprodukt führt, aus dem der Ester in einer für die Riechstoffindustrie geeigneten Qualität leicht zugänglich ist.

Die sich hieraus ergebende Aufgabe wird erfindungsgemäß entsprechend den Angaben der Patentansprüche gelöst.

Überraschenderweise ist die Synthese von Dicyclopentenylestern aus Dicyclopentadien und Carbonsäuren mit hohem Umsatz und hoher Selektivität durch Einsatz von Ionenaustauscherharzen als Katalysator möglich, wobei der Wassergehalt, bezogen auf den Reaktoreinsatz, 0,5 % bis 15 %, vorzugsweise 2 % bis 8 %, beträgt. Bei höheren Wassergehalten von über 15 % ist eine deutlich verlängerte Reaktionszeit zur Erzielung eines vergleichbaren Umsatzes erforderlich.

Zur Aufarbeitung wird das Ionentauscherharz abgetrennt, z. B. in einfacher Weise abfiltriert, und das Filtrat während der Destillation mit 5 % bis 50 %, vorzugsweise mit 10 % bis 30 % des der Carbonsäure analogen Anhydrids versetzt.

Zur Erzielung einer guten Raum-Zeit-Ausbeute setzt man, bezogen auf Dicyclopentadien, 1 bis 15 % des wasserfrei gerechneten Katalysators, vorzugsweise 2 bis 10 %, insbesondere 2 bis 5 %, ein. Bei diesen Kontaktkonzentrationen ist im allgemeinen eine Reaktionszeit von 2 bis 12 Stunden erforderlich. Bei geringeren Katalysator-Konzentrationen ist zur Erzielung des gewünschten, ca. 100 %igen Umsatzes eine wesentlich längere Reaktionszeit erforderlich. Bei einer Steigerung über 15 % hinaus ist zwar eine weitere Verringerung der Reaktionszeit festzustellen, gleichzeitig erhöht sich aber der Aufwand zur Abtrennung des Katalysators vom Rohprodukt, wenn man keine nennenswerten Verluste durch am Kontakt anhaftendes Rohprodukt verzeichnen will.

Ein wiederholter Einsatz des Ionentauscherharzes ist ebenso möglich wie die kontinuierliche oder diskontinuierliche Reaktionsführung.

Als Carbonsäuren können aliphatische und aromatische Carbonsäuren wie z. B. Essigsäure, Propionsäure, Buttersäure, Capronsäure, Isobuttersäure oder Benzoesäure eingesetzt werden. Dabei kann man sowohl die hoch konzentrierten Säuren als auch wasserhaltige Carbonsäuren verwenden. Auch der Einsatz an überschüssiger, wieder eingesetzter "Rücksäure" führt zu keiner signifikanten Umsatz-oder Selektivitätsreduzierung.

Durch Zusatz der korrespondierenden Anhydride wird die Bildung der schwer abtrennbaren störenden Zwischenläufe überraschenderweise stark reduziert. Diese Zugabe der Anhydride kann zwar auch vor dem Beginn der Fraktionierung erfolgen; durch den im Rohaustrag enthaltenen Wasseranteil ist der Verbrauch jedoch überproportional. Günstiger ist die Zugabe des entsprechenden Anhydrids nach Abtrennung der wasserhaltigen ersten Säurefraktion oder bei besonders wirksamen Destillationskolonnen nach Abtrennung des Wassers. Die korrenspondierenden Anhydride setzt man im allgemeinen in Mengen von 5 bis 50 Gew.-%, bezogen auf die eingesetzte Carbonsäure, ein, bevorzugt von 10 bis 30 % zu.

Als stark saure Ionenaustauscherharze sind beispielsweise die Lewatite SC 104, SPC 108, SPC 118 oder Amberlite XE 372 geeignet. Bei den Ionenaustauschern handelt es sich im allgemeinen um ein mit aktiven -SO₃H-Gruppen versehenes Polystyrolgrundgerüst. Bei einer Korngröße von etwa 0,3 bis 1,3 mm und einer Oberfläche von ca. 20 bis 50 m²/g besitzen diese Ionentauscherharze im allgemeinen bei einer Porosität von 20 bis 50 % eine Kapazität von 1 bis 1,5 mval/ml.

Als Reaktionstemperatur wählt man 60 bis 170 °C, vorzugsweise 100 bis 140 °C. Für den wiederholten Einsatz der Ionenaustauscher sind Reaktionstemperaturen von 100 bis 120 °C besonders geeignet. Oberhalb von 170 °C ist eine merkliche Verschlechterung der Produktqualität durch Zersetzungsprodukte und eine Zerstörung des Kontaktes festzustellen. Unterhalb von 60 °C ist neben einer zu erwartenden Verlangsamung der Reaktionsgeschwindigkeit ein vermehrter Selektivitätsverlust durch Bildung von Höhersiedern nachzuweisen.

Man kann die Reaktionskomponenten inklusive des Katalysators zu Reaktionsbeginn direkt miteinander vermischen. Günstigere Selektivitäten werden jedoch dadurch erhalten, daß man das Dicyclopentadien in die vorgelegte Carbonsäure-Katalysator-Suspension zudosiert.

Dabei empfiehlt es sich, einen molaren Überschuß an Carbonsäure, bezogen auf das Dicyclopentadien, einzusetzen. Als geeignet hat sich ein Molverhältnis von Carbonsäure zu Dicyclopentadien von 1,1 bis 5, vorzugsweise von 1,5 bis 2,5, erwiesen. Bei einem Säureunterschuß ist ein merklicher Selektivitätsverlust festzustellen. Bei größeren Säureüberschüssen als 5 wird keine nennenswerte Selektivitätssteigerung mehr festgestellt.

Die erhaltenen Ester finden in Riechstoffkombinationen mit frischen, herben und holzartig riechenden Duftkomponenten Verwendung. Die Riechstoffkombinationen setzt man in Seifen, Waschmitteln und Deodorants ein.

Beispiele <u>1</u> bis <u>8</u>

Als Standard-Ansatz wird folgende Rezeptur gewählt:

4,5 Mol der Carbonsäure werden mit 13,8 g des jeweiligen wasserfeuchten Ionentauscherharzes (wasserfrei gerechnet) in einem Rührkolben mit aufgesetztem Kühler dispergiert. Nach Temperieren auf die in der nachfolgenden Tabelle angegebene Temperatur werden innerhalb von 4 h 3,0 Mol (396 g) Dicyclopentadien (DCP) zudosiert, eine Stunde nachgerührt und danach der Katalysator gut abfiltriert. (Das Molverhältnis von Carbonsäure zu Dicyclopentadien beträgt 1,5.) Bezogen auf den gesamten Reaktoreinsatz wurde jeweils ein Wassergehalt von 3,5 % eingestellt.

Zur Destillation wird zunächst beispielsweise bei 200 mbar an einer 0,5 m Multifilkolonne vornehmlich das Wasser abgetoppt. Nach Zugabe von 0,6 Mol des Carbonsäureanhydrids wird die Destillatabnahme kurz unterbrochen, um danach die überschüssige Säure nebst Anhydrid abzutoppen. Bei einem Vakuum von 2 mbar wird danach ein möglichst geringer Zwischenlaufanteil abgenommen, um sodann den Hauptlauf abtrennen zu können.

3

| Beisp. | Katalysator | Carbonsäure bzw. Carbonsäureanhydrid | Reaktions-temperatur | Umsatz | Selektivität |
|--------|-------------|-------------------|----------------------|--------|--------------|
| | | | | bezogen auf DCP (%) | |
| 1 | Lewatit SC 104 | Essigsäure | ca. 125 °C | 99 | 73 |
| 2 | Lewatit SPC 108 | Essigsäure | ca. 125 °C | 100 | 92 |
| 3 | Lewatit SPC 118 | Essigsäure | ca. 110 °C | 100 | 85 |
| 4 | Amberlite XE 372 | Essigsäure | ca. 125 °C | 99 | 82 |
| 5 | Lewatit SPC 108 | Propionsäure | ca. 140 °C | 100 | 87 |
| 6 | Lewatit SPC 108 | Buttersäure | ca. 140 °C | 100 | 88 |
| 7 | Lewatit SPC 108 | Isobuttersäure | ca. 125 °C | 100 | 69 |
| 8 | Lewatit SPC 108 | Benzoesäure | ca. 130 °C | 85 | 70 |

Vergleichsbeispiel A

Im Vergleich zu den wasserfeuchten Katalysatoren wird handelsüblicher trockener Katalysator eingesetzt. Die Ergebnisse sind identisch mit den Werten, die nach Trocknung eines wasserfeuchten Katalysators durch Auskreisen mit Cyclohexan erzielt werden. Zur Erzielung eines in etwa vergleichbaren Umsatzes muß eine Verlängerung der Reaktionszeit nach Zugabe des DCP's auf ca. 20 h in Kauf genommen werden.

| Vergl.-Beisp. | Katalysator | Carbonsäure | Reaktions-temperatur | Umsatz (%) | Selektivität (%) |
|---------------|-------------|-------------|----------------------|------------|------------------|
| A | Lewatit SPC 108 | Essigsäure | 125 °C | 85 | 54 |

Beispiel 9

Bei Einsatz von wiedereingesetzter Rückessigsäure mit wasserfeuchtem Lewatit SPC 108 wurde bei einem Umsatz von 100 % eine Selektivität von 85 % erzielt.

Beispiele 10 bis 13

Der Einfluß der Wasserkonzentration, bezogen auf den Reaktoreinsatz unter den Bedingungen des Standard-Ansatzes nach Beispiel 1 bis 8, ist aus der folgenden Tabelle zu entnehmen. Als Carbonsäure fungierte Essigsäure und als Katalysator Lewatit SPC 108.

4

| Bei-spiel | Reaktions-temperatur (°C) | Zeit (h) | Wassergehalt (%) | Umsatz (%) | Selektivität (%) |
|---|---|---|---|---|---|
| 10 | 125 | 12 | 0 | 71 | 55 |
| 11 | 125 | 2 | 0,7 | 92 | 65 |
| 12 | 125 | 5 | 3,5 | 100 | 92 |
| 13 | 125 | 9 | 8 | 98 | 89 |
| 14 | 125 | 11 | 15 | 99 | 86 |

Beispiele 14 und 15

Der Einfluß der Kontaktkonzentration unter den Bedingungen des Standard-Ansatzes nach Beispiel 1 bis 8, berechnet als wasserfreie Substanz und bezogen auf Dicyclopentadien, ist aus folgender Tabelle zu entnehmen. Als Carbonsäure fungierte Essigsäure und als Katalysator Lewatit SPC 108.

| Bei-spiel | Reaktions-temperatur (°C) | Zeit (h) | Kontakt Konzentration g/mol DCP | Umsatz (%) | Selektivität (%) |
|---|---|---|---|---|---|
| 15 | 125 | 7 | 2,5 | 100 | 81 |
| 16 | 125 | 2,5 | 15,0 | 100 | 84 |

**Ansprüche**

1. Verfahren zur Herstellung von Dicyclopentenylestern durch Addition von Carbonsäuren an Dicyclopentadien in Gegenwart von stark sauren Ionenaustauscherharzen,
dadurch gekennzeichnet,
daß man Ionenaustauscherharze einsetzt, wobei der Wassergehalt, bezogen auf den Reaktoreinsatz, 0,5 bis 15 % beträgt, ein Molverhältnis von Carbonsäure zu Dicyclopentadien von 1,1 bis 5 einstellt, die Umsetzung bei einer Temperatur von 60 bis 170 °C durchführt und bei bzw. vor der anschließenden destillativen Aufarbeitung die korrespondierenden Anhydride zusetzt.

2. Verfahren nach Anspruch 1,
dadurch gekennzeichnet,
daß man mit Wassergehalten im Reaktoreinsatz von 2 bis 8 % arbeitet.

3. Verfahren nach Anspruch 1 und 2,
dadurch gekennzeichnet,
daß man ein Molverhältnis von Carbonsäure zu Dicyclopentadien von 1,5 bis 2,5 einstellt.

4. Verfahren nach einem der Ansprüche 1 bis 3,
dadurch gekennzeichnet,
daß man die Umsetzung bei Temperaturen von 100 bis 140 °C durchführt.

5. Verfahren nach einem der Ansprüche 1 bis 4,
dadurch gekennzeichnet,
daß man den Ionenaustauscher in einer Konzentration von 1 bis 15 %, bevorzugt 2 bis 10 %, wasserfrei gerechnet, einsetzt.

6. Verfahren nach einem der Ansprüche 1 bis 5,
dadurch gekennzeichnet,
daß man bei bzw. vor der destillativen Aufarbeitung die korrespondierenden Anhydride in Mengen von 5 bis 50 Gew.-%, bezogen auf die eingesetzte Carbonsäure, bevorzugt in Mengen von 10 bis 30 % zusetzt.